# EUROPEAN PATENT SPECIFICATION

(11) **EP 3 442 424 B1**
(45) Date of publication and mention of the grant of the patent: **07.10.2020**
(21) Application number: 17736785.1
(22) Date of filing: 10.04.2017
(51) Int. Cl.: A61B 6/02, A61B 6/00

(54) **MOBILE TOMOSYNTHESIS SYSTEM AND METHOD**
SYSTEM UND VERFAHREN ZUR MOBILEN TOMOSYNTHESE
SYSTÈME ET PROCÉDÉ DE TOMOSYNTHÈSE MOBILE

(30) Priority: 13.04.2016 US 201662321766 P
(43) Date of publication of application: 20.02.2019
(73) Proprietor: Carestream Health, Inc., Rochester, NY 14608 (US)
(72) Inventor: HEATH, Michael D., Rochester NY 14608 (US); SNYDER, William F., Rochester NY 14608 (US); BATURIN, Pavlo, Rochester NY 14608 (US)
(74) Representative: Schmidbauer, Andreas Konrad
(86) International application number: PCT/US2017/026783
(87) International publication number: WO 2017/180507

(56) References cited:
- DE-A1- 1 914 393
- DE-A1- 3 036 195
- US-A1- 2007 223 650
- US-A1- 2011 210 261
- US-A1- 2014 093 032
- US-A1- 2014 369 459

## Description

### FIELD OF THE INVENTION

The present invention relates generally to the field of radiography and in particular to portable radiographic imaging systems. More specifically, the invention relates to mobile radiography systems and methods for undertaking bedside tomographic imaging.

### BACKGROUND

Mobile x-ray systems are of particular value in intensive care unit (ICU) and other environments where timely acquisition of a radiographic image is important. Because it can be wheeled around the ICU or other area and brought directly to the patient's bedside, a mobile x-ray system allows an attending physician or clinician to have recent information on the condition of a patient and helps to reduce the risks entailed in moving patients to stationary equipment in the radiological facility.

Tomosynthesis image capture involves taking several projection images with x-ray focal spots positioned at different relative angles with respect to an imaged subject. In-room tomosynthesis systems have a detector and a tube crane built into an imaging room facility to move an x-ray tube with a stable motion. A portable or mobile system places the x-ray source at the end of a boom or support arm attached to a movable transport cart, which may lead to an unstable system when the x-ray tube is moved during an imaging sequence.

One concern that must be addressed in design of the support arm relates to ease of positioning of the x-ray source mounted on its boom. For ease of operation under varying conditions, the technician should be able to easily position and orient the x-ray source without the need of additional tools and without needing help from nearby personnel. This includes moving the x-ray source from its docked position used in transport to an imaging position. The mechanical problem of providing ease of positioning is complicated by the weight of the x-ray source and by its extension outward from the base of the mobile imaging system.

US 2014 093 032 A1 discloses a configuration and method for tomosynthetic fluoroscopy containing a rotatable mounting device with an x-ray emitter rotating about an optical axis such that a focus of the x-ray emitter describes a circular path.

DE 19 14 393 A1 discloses a tomograph which consists of an upper frame carrying an X-ray tube, and a lower support frame which supports a film carrier. The X-ray tube can be moved in the upper frame, and the upper frame has a counterweight being provided in a kinematic connection which ensures weight compensation.

US 2011 210 261 A1 discloses a barrel-style rotatable gantry structure. A radiation treatment head and a guidance system including an x-ray source are mounted to a rotatable gantry structure in a manner that facilitates rotation around a rotation axis while keeping the rotation axis stationary.

DE 30 36 195 A1 discloses a transport mechanism using a counterweight moved in synchronism with the forward and return movement of an imaging film cassette. A conversion ratio between the transport mechanism and a drive is varied in dependence on the size of the x-ray cassette being handled.

US 2014 223 650 A1 discloses a support structure having an axis of rotation and a pair of first and second radiation modules fixedly arranged thereon along a radius perpendicular to the axis of rotation. Each of the radiation modules comprises a radiation source simultaneously emitting radiation and a radiation detector for detecting radiation as emitted from the radiation modules and passed through an object being imaged.

Thus, there is a need for improvements in mobile x-ray system design that allow these devices to be more easily transported and deployed.

### SUMMARY OF THE INVENTION

In accordance with the present invention, a linear counterbalance apparatus and a method of capturing tomographic images as set forth in Claims 1 and 3, respectively, is provided. Further embodiments of the invention are inter alia disclosed in the dependent claims.

Disclosed herein is a weight system to maintain the center of mass of the tube head, or the x-ray source assembly, stationary during imaging sequence x-ray tube movement. The system counterbalances the weight and, in some embodiments, keeps the system stable by countering the forces applied while starting and stopping the tube motion. In a linear embodiment, the weight balance is provided because the center of mass as between the x-ray tube/collimator assembly and the counterweight remains fixed, because the x-ray tube/collimator assembly and the counterweight are directed in opposite directions at the start and stop of tube motion. The linear tube motion with two spatially separated counterweights places the center of mass of the x-ray tube/collimator on the same line (as it is moved) as the center of mass of the two counterweights. Thus, an x-ray tube may be stably attached to, and used in motion, at the end of a boom, or support arm.

In one embodiment, an x-ray source assembly includes an x-ray source mechanism configured to move an x-ray source along an imaging trajectory, and a counterweight is moved simultaneously with the x-ray source to counterbalance the x-ray source as the x-ray source moves.

In one embodiment, an x-ray source assembly is moved along an imaging trajectory in a first direction. An x-ray source in the x-ray source assembly is fired multiple times during the step of moving the x-ray source to capture radiographic images of an object. A counterweight is moved simultaneously with the x-ray source to counterbalance a weight of the x-ray source.

This brief description of the invention is intended only to provide a brief overview of subject matter disclosed herein according to one or more illustrative embodiments, and does not serve as a guide to interpreting the claims or to define or limit the scope of the invention, which is defined only by the appended claims. This brief description is provided to introduce an illustrative selection of concepts in a simplified form that are further described below in the detailed description. This brief description is not intended to identify key features or essential features of the claimed subject matter, nor is it intended to be used as an aid in determining the scope of the claimed subject matter. The claimed subject matter is not limited to implementations that solve any or all disadvantages noted in the background.

### BRIEF DESCRIPTION OF THE DRAWINGS

So that the manner in which the features of the invention can be understood, a detailed description of the invention may be had by reference to certain embodiments, some of which are illustrated in the accompanying drawings. It is to be noted, however, that the drawings illustrate only certain embodiments of this invention and are therefore not to be considered limiting of its scope, for the scope of the invention encompasses other equally effective embodiments. The drawings are not necessarily to scale, emphasis generally being placed upon illustrating the features of certain embodiments of the invention. In the drawings, like numerals are used to indicate like parts throughout the various views. Thus, for further understanding of the invention, reference can be made to the following detailed description, read in connection with the drawings in which:
FIG. 1A is a perspective view of an exemplary linear counterbalance for an x-ray assembly of an x-ray imaging system;
FIG. 1B is a schematic side view diagram of FIG. 1A;
FIG. 1C is a schematic top view diagram of FIG. 1A;
FIG. 1D is a schematic side view diagram of FIG. 1A showing translation and rotation of the x-ray assembly;
FIG. IE is a schematic side view of an exemplary arcuate counterbalance for an x-ray assembly of an x-ray imaging system;
FIG. 2A is a perspective view of an exemplary unclaimed arc counterbalance for an x-ray source of an x-ray imaging system;
FIG. 2B is a schematic side view diagram of FIG. 2A;
FIG. 3 is a diagram of an exemplary unclaimed circular counterbalance for x-ray sources of an x-ray imaging system; and
FIGS. 4A-4B are perspective views of an exemplary mobile radiography system using the counterbalance apparatuses disclosed herein.

### DETAILED DESCRIPTION OF THE INVENTION

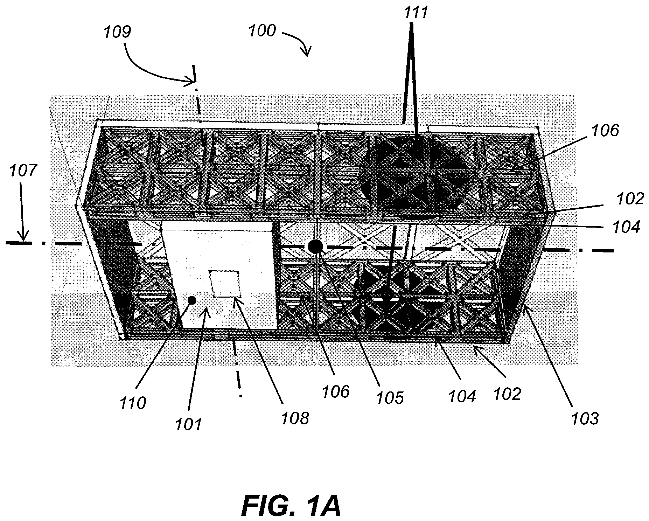

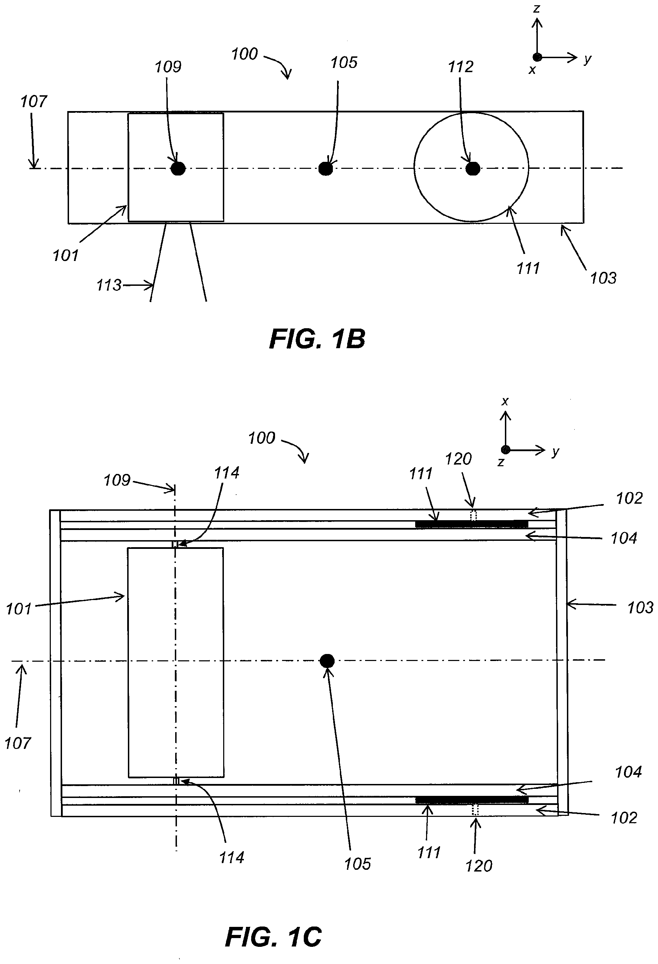

As shown in FIGS. 1A - 1C, a linear counterbalance apparatus 100 comprises an x-ray source assembly 101 secured within a rigid rectangular shaped frame 103. The rectangular shaped frame 103 may be said to have a width along the x dimension, a length along the *y* dimension, and a depth along the z dimension. The frame 103 may include two pairs of similarly constructed sidewalls 102, 104, along the length dimension, shown as having a lattice construction in FIG. 1A. Each pair of sidewalls may be said to have an outer sidewall 102 and an inner sidewall 104, and operate in a similar manner as described herein. One pair of the sidewalls 102, 104, used for exemplary description herein, is shown positioned adjacent opposite sides of one of a pair of circular shaped counterweights 111. The outer sidewalls 102 may include a central rail 106 to which a central support for the counterweights 111 may be attached. The central support for the counterweights 111 may include a pin or rod 120 extending through a center of the counterweight 111. The central rails 106 of the outer sidewalls 102 may each include a track for moving the counterweights 111 simultaneously back and forth along a linear path parallel to a central axis 107 of the frame 103. The tracks in the central rails 106 may be formed as chains having an orifice to receive the pins or rods 120 acting as the central support for the counterweights 111. The chains may be motorized to move back-and-forth within the central rail 106 simultaneously, thereby moving the counterweights 111 back-and-forth simultaneously as desired. The inner sidewalls 104 may also be similarly constructed as the outer sidewalls 102, such as by each having a central rail 106 with a chained track to support and move the counterweights 111, such as by receiving an opposite end of the central support rod or pin 120 of the counterweights 111. Alternatively, only the outer sidewalls 102 may be configured to provide support for, and a chained track for moving, the counterweights 111, while the inner sidewalls 104 may provide incidental contact support as the counterweights 111 move along the length of the frame 103. In another embodiment, a central rail of the inner sidewalls 104 may be slotted along a length of the inner sidewalls 104 to passively receive and support an opposite end of the support rod or pin 120 of the counterweight 111 as it is driven by the chained track within the central rail 106 of the outer sidewall 102.

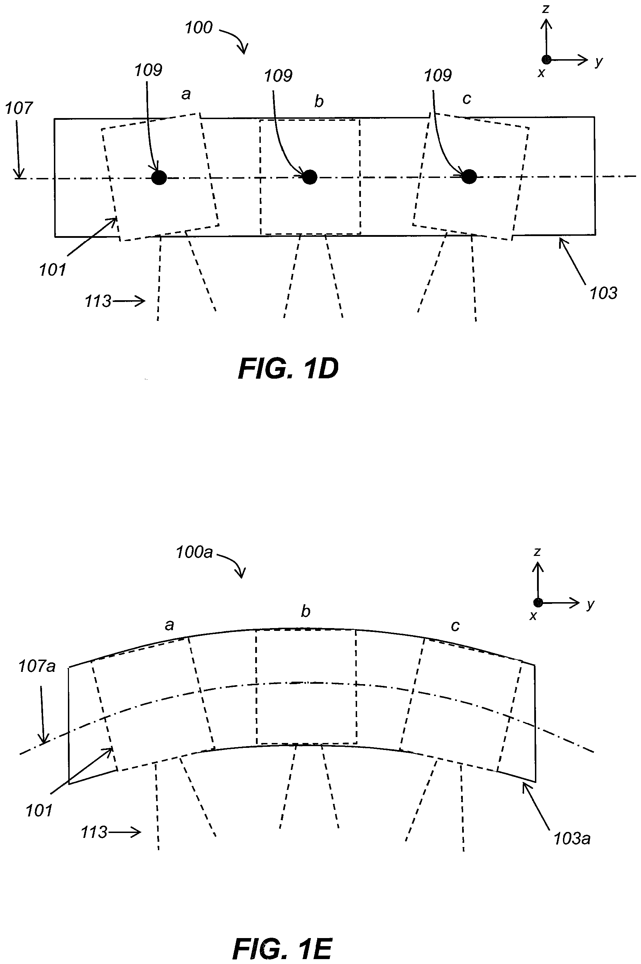

The x-ray source assembly 101 may include a housing 110 enclosing an x-ray source having a focal spot coinciding with rotational axis 109. The x-ray source assembly 101 may include a collimator 108 on its bottom side for shaping an x-ray beam 113 from x-rays emitted by an x-ray source in the x-ray assembly 101. The x-ray source assembly 101 may be movably attached to inner sidewalls 104 for moving and rotating the x-ray source while the x-ray source is fired during a tomographic imaging sequence. The x-ray source assembly 101 may include support rods 114 extending therefrom and attached to inner sidewalls 104. The inner sidewalls 104 may include central rails 106 similar to the central rails 106 of the outer sidewalls 102 to secure and move the support rods 114 along a length of the frame 103, thereby moving the x-ray source of the x-ray assembly 101 along a length of the frame 103. One or both of the inner sidewalls 104 may further include a mechanism attached to one or both of the support rods 114 to rotate the x-ray assembly 101 about rotational, or tilt, axis 109 while it is moved along the length of the frame 103, as illustrated in FIG. 1D. The x-ray assembly 101 may be rotated about the rotational axis 109 so that the x-ray beam 113 emitted by the x-ray source may be properly aimed toward an x-ray detector and an object to be exposed by the x-ray beam 113 as the x-ray assembly 101 is moved along the length the frame 103 during a tomographic imaging sequence, such as from position *a,* to position *b*, to position *c*, as illustrated in FIG. 1D. Although three exemplary positions *a*, *b*, *c*, are illustrated in FIG. ID, x-ray assembly 101 may be translated along a path parallel to central axis 107 of the frame 103 and tilted about rotational axis 109 continuously during a tomographic imaging sequence, and the x-ray source therein may be fired 30 times, 60 times, or more, as the x-ray assembly 101 moves from a starting position, such as position *a*, to a terminal position, such as position *c.*

The box assembly, or frame, 103 may be positioned at any location and any angle prior to the start of a tomographic imaging sequence. In one embodiment, the x-ray assembly 101 and the counterweights 111 are positioned as shown in FIG. 1B at the start of a tomographic imaging sequence, although the x-ray assembly 101 and the counterweights 111 may be positioned as desired. The center of mass 105 of the linear counterbalance apparatus 100 may be maintained in a stationary spatial location relative to the counterbalance apparatus 100 so long as the x-ray assembly 101 and the counterweights 111 move simultaneously in opposite directions along paths parallel to the central axis 107 of the frame 103. Depending on their relative weight difference, the x-ray assembly 101 and the counterweights 111 may be moved at different speeds. If the total weight of the two counterweights 111 is selected to be approximately equal to a weight of the x-ray assembly 101, then the x-ray assembly 101 and the counterweights 111 may be moved at approximately the same speed in opposite directions relative to, and parallel with, the central axis 107 of the frame 103. The center of mass 105 of the x-ray source assembly 101 and counterweights 111 may follow a linear trajectory along central axis 107 if the x-ray source assembly 101 or counterweights 111 are moved individually. The center of mass of the counterweights 111 is located along a line between the counterweights 111, and may move collinearly with the movement of the center of mass of x-ray source assembly 101. The center of mass of the counterweights 111 and may not necessarily move collinearly with the movement of the center of mass of x-ray source assembly 101 if their weights are not symmetrically disposed within the frame 103, but their centers of mass move along parallel lines. The counterbalance apparatus 100 may be designed symmetrically so that the centers of mass 105 of the x-ray assembly 101 and counterweights 111 are collinear with the central axis 107 of the frame 103, as shown in FIG. 1A.

FIG. IE illustrates an embodiment of an arcuate counterbalance apparatus 100a whereby the frame 103a structure is similar to the frame structure 103 disclosed herein except that the frame 103a includes a curved profile, as shown in FIG. 1E, rather than rectangular. In this embodiment, the curvature of the frame 103a may be selected such that the x-ray assembly 101 may be moved along rails of the frame 103a, as described herein, during a tomographic imaging sequence such that the curvature of the frame 103a serves to aim the x-ray beam 113 emitted by the x-ray source in the x-ray assembly 101 toward an object to be tomographically imaged without requiring an independent rotation of the x-ray assembly 101 about an axis, such as axis 109, disclosed herein.

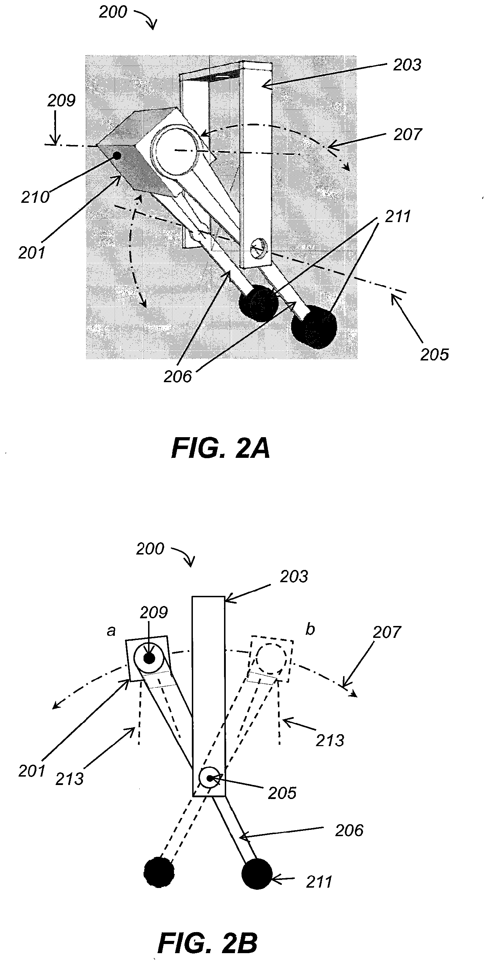

As shown in FIGS. 2A-B, an unclaimed arc counterbalance apparatus 200 comprises an x-ray source assembly 201 rotatably attached on its opposite sides to two rigid arms 206 extending therefrom. The two arms 206, in turn, are each rotatably secured to a three-sided U-bracket 203. At the distal ends of the arms 206 are secured counterweights 211. The x-ray assembly 201 may include a collimator (not shown) similar to the x-ray and collimator assembly of FIG. 1A to shape x-rays emitted from an x-ray source within the x-ray assembly 201 into a desired x-ray beam 213. A focal spot of the x-ray source within the x-ray assembly 201 may coincide with a rotational axis 209 of the x-ray assembly 201. Altogether, the x-ray assembly 201, the arms 206, and the counterweights 211 may be configured to rotate about the axis 205 coinciding with the U-bracket 203 so that the x-ray assembly 201 is translated along the angular path 207, shaped as an arc centered at axis 205, while the x-ray assembly 201 itself rotates with respect to the arms 206 and the rotational axis 209 so that the x-ray beam 213 is aimed (FIG. 2B) toward an x-ray detector and an object to be tomographically imaged while the x-ray source is fired during a tomographic imaging sequence.

The x-ray source assembly 201 may include a housing 210 enclosing an x-ray source having its focal spot coinciding with rotational axis 209. The arms 206 may be configured to receive support rods extending from the x-ray assembly 201 similar to the support rods 114 of the x-ray assembly 101. One or both of the arms 206 may be configured with a mechanism to rotate the x-ray assembly 201 about axis 209 at a rate corresponding to a rate of translation of the x-ray assembly 201 about axis 205 during a tomographic imaging sequence. The counterweights 211 may have a weight selected to counterbalance the weight of the x-ray assembly 201 and the arms 206 such that a center of mass of the x-ray assembly 201, the arms 206, and the counterweights 211 coincides with the axis 205. The center of mass of the system is maintained during an imaging sequence translation of the x-ray source assembly 201 and counterweights 211. Counterweights 211 move in the same angular direction as the movement of the x-ray source assembly 201 to maintain a stationary spatial position of the center of mass of the entire structure to coincide with the axis 205. The center of mass of the two counterweights 211 is between the counterweights 211.

The x-ray assembly 201 may be rotated about the rotational axis 209 (FIG. 2B) so that the x-ray beam 213 emitted by the x-ray source in the x-ray assembly 201 may be properly aimed toward an x-ray detector and an object to be exposed by the x-ray beam 213 as the x-ray assembly 201 is translated along the angular path 207 during a tomographic imaging sequence, such as from position *a* to position *b* as illustrated in FIG. 2B. Although two exemplary positions *a*, *b*, are illustrated in FIG. 2B, x-ray assembly 201 may be translated along the angular path 207 centered about the axis 205 continuously during a tomographic imaging sequence, and the x-ray source therein may be fired 30 times, 60 times, or more, as the x-ray assembly 201 moves from a starting position, such as position *a*, to a terminal position, such as position b.

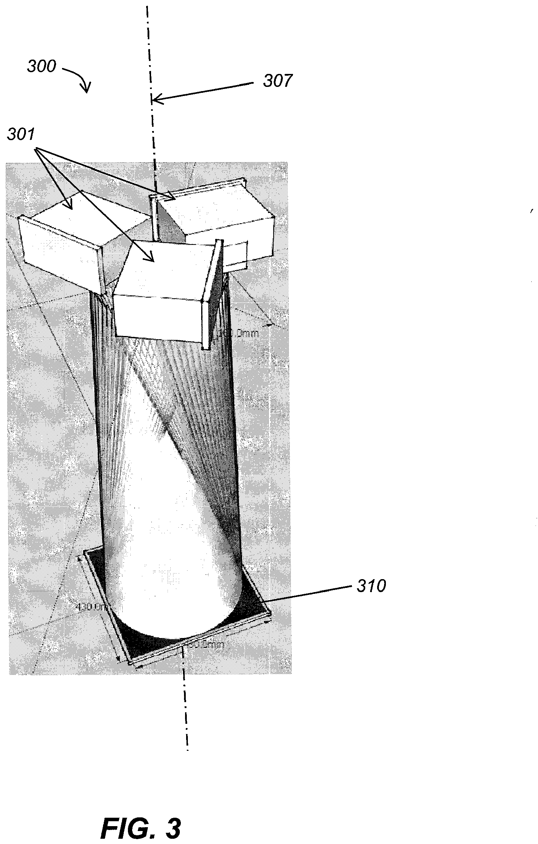

As shown in FIG. 3, an unclaimed circular counterbalance system 300 comprises a plurality of x-ray sources 301 configured to rotate about a central axis 307. Each x-ray source 301 is configured to rotate about the axis 307 an equal distance along an arc path in a common plane. A curvature of the arc paths of the plurality of x-ray sources 301 may circumscribe a circular arc whose projection includes a cylindrical shape projected onto a detector 310, which cylindrical projection is symmetrically positioned with respect to edges of the detector 310. The detector 310 is positioned below the x-ray sources 301 to receive x-rays emitted by the x-ray sources 301 aimed toward the detector 310. An object to be radiographically imaged may be placed therebetween such that the detector 310 captures the radiographic images of the object when any one or more of the x-ray source 301 are fired.

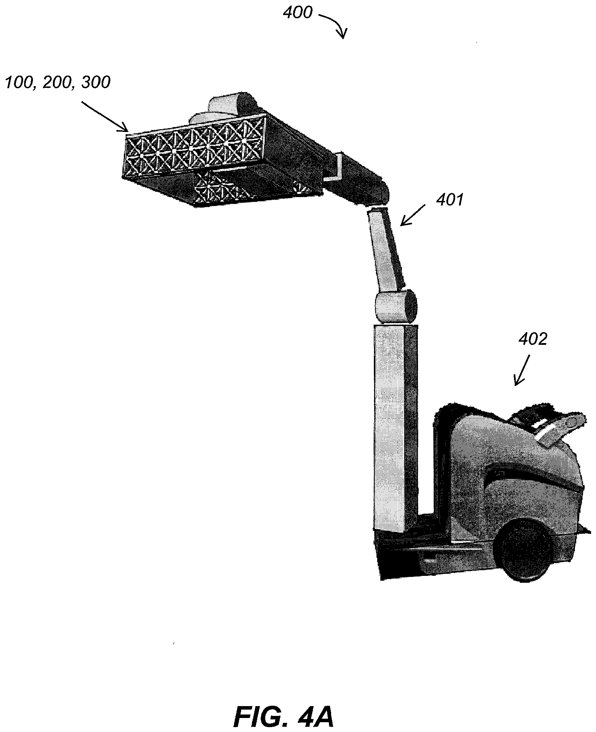

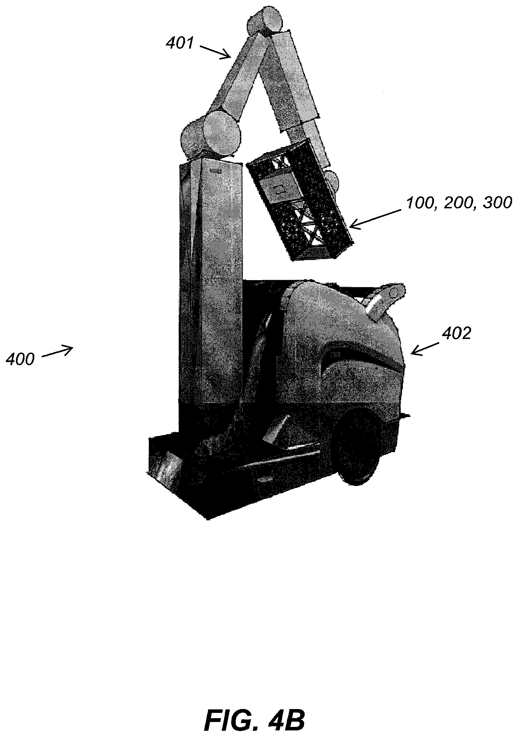

FIGS. 4A-4B illustrate a mobile, bedside tomosynthesis system 400 including a linear counterbalance apparatus 100 attached to one end of a segmented arm 401 of the bedside tomosynthesis system 400. Another end of the segmented arm 401 is, in turn, attached to a wheeled, transportable mobile base 402 which may be rolled across surfaces to bring the bedside tomosynthesis system 400 to patient rooms in a medical facility, as desired. The arm 401 may be manipulated by motor control or manually to position the counterbalance apparatus 100, 200, 300 at any 3D spatial angle and position. FIG. 4B illustrates the mobile, bedside tomosynthesis system 400 arranged in a stowed transport position for rolling along a floor. After an imaging sequence commences, the counterbalance apparatus 100, 200, 300, maintains a constant center of mass, as described herein, so that the entire counterbalance apparatus 100, 200, 300 does not move with respect to the object to be imaged.

Although FIGS. 4A-B illustrate the linear counterbalance apparatus 100 embodiment attached to one end of the arm 401, any of the embodiments described herein may be attached to the arm 401 to enable the bedside tomosynthesis system 400 to provide a variety of mobile tomosynthesis imaging embodiments. The arm 401 of the mobile, bedside tomosynthesis system 400 may be embodied in various forms. The arm 401 may include one or more elbow joints and/or universal joints; the arm 401 may include telescoping linear sections; the arm 401 may include a vertical column, telescoping or otherwise vertically height adjustable, and a horizontal boom rotatably coupled to the column, which boom may also be telescoping or otherwise horizontally extendable; or a combination thereof. Suitable mobile x-ray imaging systems that may be fitted with the x-ray counterbalance apparatuses 100, 200, 300, disclosed herein include those described in US 8 961 011 B2, issued February 24, 2015, to Lalena, entitled MOBILE RADIOGRAPHY UNIT HAVING MULTIPLE MONITORS; US 8 568 028 B2, issued October 29, 2013, to Wendlandt, et al., entitled MOBILE RADIOGRAPHY UNIT HAVING COLLAPSIBLE SUPPORT COLUMN; and US 8 672 543 B2, issued March 18, 2014, to Kralles, et al., entitled COUNTERWEIGHT FOR MOBILE X-RAY DEVICE.

In another embodiment, a tomosynthesis imaging system includes a transportable, rollable base, a segmented support arm attached to the base, and an x-ray source assembly attached to the support arm, wherein the x-ray source assembly is configured to move along an imaging trajectory. A counterweight is configured to move in concert with the x-ray source assembly to counterbalance the x-ray source assembly as the x-ray source assembly moves along the imaging trajectory. The support arm may comprise a first end attached to the movable base and a second end attached to the x-ray source assembly. The imaging trajectory may be selected from the trajectories consisting of a circular arc, linear, and arcuate. The x-ray source structure may comprise a plurality of x-ray sources each configured to move along at least a portion of the imaging trajectory, wherein the x-ray sources are each configured to counterbalance remaining ones of the x-ray sources as the x-ray sources move along said at least a portion of an imaging trajectory. A spatial location of a center of mass of the x-ray source structure remains substantially unchanged as the x-ray source and the counterweights moves along their respective trajectories. The counterweight may comprise at least two masses spaced apart on opposite sides of a center of mass trajectory. The movement of the counterweights traverses a path having the same shape at the imaging trajectory.

As used herein, the term "x-ray source assembly" may include a collimator, along with other moving components. In one embodiment, a mobile tomosynthesis system uses an x-ray source assembly attached to a support arm which, in turn, is attached to a movable base. The x-ray source assembly is configured to move the x-ray source along an imaging trajectory, and a counterweight is configured to move to counterbalance the x-ray source assembly as the x-ray source moves along the imaging trajectory.

These objects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

## Claims

1. A linear counterbalance apparatus (100) comprising:
an x-ray source mechanism configured to move an x-ray source assembly (101) along a linear imaging trajectory in a first direction; and
a counterweight (111) configured to move in a second direction opposite the first direction simultaneously with the x-ray source assembly (101) to counterbalance the x-ray source assembly (101) as the x-ray source assembly (101) moves along the imaging trajectory;
**characterized in that**
the counterweight (111) comprises at least two masses spaced apart; and
the counterweight (111) is configured to move such that the center of mass of the counterweight (111) is located along a line between the at least two masses and moves collinearly with a movement of the center of mass of the x-ray source assembly (101) as the x-ray source assembly (101) moves along the imaging trajectory.

2. The apparatus (100) of claim 1, wherein a spatial location of the center of mass of the x-ray source assembly (101) and the counterweight remains substantially unchanged as the x-ray source assembly (101) and counterweight (111) move.

3. A method of capturing tomographic images, the method comprising:
moving an x-ray source assembly (101) along a linear imaging trajectory in a first direction;
firing an x-ray source in the x-ray source assembly (101) multiple times during the step of moving the x-ray source assembly (101) to capture radiographic images of an object; and
moving a counterweight (111) in a second direction opposite the first direction, simultaneously with the step of moving the x-ray source assembly (101) to counterbalance the x-ray source assembly (101);
**characterized in that** the step of moving the counterweight (111) further comprises:
moving at least two masses spaced apart; and
moving the center of mass of the counterweight (111) along a line between the at least two masses, collinearly with a movement of the center of mass of the x-ray source assembly (101) as the x-ray source assembly (101) moves along the imaging trajectory.

4. The method of claim 3, further comprising maintaining the center of mass of the x-ray source assembly (101) and the counterweight (111) spatially stationary during the steps of moving the x-ray source assembly (101) and moving the counterweight (111).

5. The method of claim 3, further comprising rotating the x-ray source assembly (101) simultaneously with the step of moving the x-ray source assembly (101) along the linear imaging trajectory.

## Patentansprüche

1. Eine lineare Gegengewichts- bzw. Gewichtsausgleichsvorrichtung (100), die Folgendes aufweist:
einen Röntgenstrahlquellenmechanismus, der eingerichtet ist, eine Röntgenstrahlquellenanordnung (101) entlang einer linearen Bildgebungsbahn in eine erste Richtung zu bewegen; und
ein Gegen- bzw. Ausgleichsgewicht (111), das eingerichtet ist, sich gleichzeitig mit der Röntgenstrahlquellenanordnung (101) in eine zweite Richtung entgegengesetzt zur ersten Richtung zu bewegen, um die Röntgenstrahlquellenanordnung (101) auszubalancieren bzw.
auszugleichen, wenn sich die Röntgenstrahlquellenanordnung (101) entlang der Bildgebungsbahn bewegt;
**dadurch gekennzeichnet, dass**
das Gegengewicht (111) mindestens zwei voneinander beabstandete Massen aufweist; und
das Gegengewicht (111) eingerichtet ist, sich so zu bewegen, dass sich der Massenschwerpunkt des Gegengewichts (111) entlang einer Linie zwischen den mindestens zwei Massen befindet und sich kollinear mit einer Bewegung des Massenschwerpunkts der Röntgenstrahlquellenanordnung (101) bewegt, wenn sich die Röntgenstrahlquellenanordnung (101) entlang der Bildgebungsbahn bewegt.

2. Die Vorrichtung (100) nach Anspruch 1, wobei eine räumliche Lage des Massenschwerpunkts der Röntgenstrahlquellenanordnung (101) und des Gegengewichts im Wesentlichen unverändert bleibt, wenn sich die Röntgenstrahlquellenanordnung (101) und das Gegengewicht (111) bewegen.

3. Ein Verfahren zum Aufnehmen tomographischer Bilder, wobei das Verfahren Folgendes aufweist:
Bewegen einer Röntgenstrahlquellenanordnung (101) entlang einer linearen Bildgebungsbahn in eine erste Richtung;
mehrmaliges Abfeuern einer Röntgenstrahlenquelle in der Röntgenstrahlquellenanordnung (101) während des Schritts des Bewegens der Röntgenstrahlquellenanordnung (101), um radiographische Bilder eines Objekts aufzunehmen; und
Bewegen eines Gegen- bzw. Ausgleichsgewichts (111) in eine zweite Richtung entgegengesetzt zur ersten Richtung, gleichzeitig mit dem Schritt des Bewegens der Röntgenstrahlquellenanordnung (101), um die Röntgenstrahlquellenanordnung (101) auszubalancieren bzw.
auszugleichen;
**dadurch gekennzeichnet, dass** der Schritt des Bewegens des Gegengewichts (111) ferner Folgendes aufweist:
Bewegen von mindestens zwei Massen, die voneinander beabstandet sind; und
Bewegen des Massenschwerpunkts des Gegengewichts (111) entlang einer Linie zwischen den mindestens zwei Massen, und zwar kollinear mit einer Bewegung des Massenschwerpunkts der Röntgenstrahlquellenanordnung (101), wenn sich die Röntgenstrahlquellenanordnung (101) entlang der Bildgebungsbahn bewegt.

4. Das Verfahren nach Anspruch 3, das weiterhin Folgendes aufweist:
Halten des Massenschwerpunkts der Röntgenstrahlquellenanordnung (101) und des Gegengewichts (111) räumlich stationär während der Schritte des Bewegens der Röntgenstrahlquellenanordnung (101) und des Bewegens des Gegengewichts (111).

5. Das Verfahren nach Anspruch 3, das ferner Drehen der Röntgenstrahlquellenanordnung (101) gleichzeitig mit dem Schritt des Bewegens der Röntgenstrahlquellenanordnung (101) entlang der linearen Bildgebungsbahn aufweist.

## Revendications

1. Appareil à contrepoids linéaire (100) comprenant :
un mécanisme de source de rayons X configuré pour déplacer un assemblage de source de rayons X (101) le long d'une trajectoire d'imagerie linéaire dans une première direction ; et
un contrepoids (111) configuré pour se déplacer dans une deuxième direction opposée à la première direction simultanément avec l'assemblage de source de rayons X (101) pour contrebalancer l'assemblage de source de rayons X (101) quand l'assemblage de source de rayons X (101) se déplace le long de la trajectoire d'imagerie ;
**caractérisé en ce que**
le contrepoids (111) comprend au moins deux masses espacées ; et
le contrepoids (111) est configuré pour se déplacer de sorte que le centre de masse du contrepoids (111) est situé le long d'une ligne entre lesdites au moins deux masses et se déplace colinéairement avec un déplacement du centre de masse de l'assemblage de source de rayons X (101) quand l'assemblage de source de rayons X (101) se déplace le long de la trajectoire d'imagerie.

2. Appareil (100) selon la revendication 1, dans lequel un emplacement spatial du centre de masse de l'assemblage de source de rayons X (101) et du contrepoids demeure sensiblement inchangé quand l'assemblage de source de rayons X (101) et le contrepoids (111) se déplacent.

3. Procédé de capture d'images tomographiques, le procédé comprenant les étapes suivantes :
le déplacement d'un assemblage de source de rayons X (101) le long d'une trajectoire d'imagerie linéaire dans une première direction ;
le déclenchement d'une source de rayons X dans l'assemblage de source de rayons X (101) à plusieurs reprises pendant l'étape de déplacement de l'assemblage de source de rayons X (101) pour capturer des images radiographiques d'un objet ; et
le déplacement d'un contrepoids (111) dans une deuxième direction opposée à la première direction, simultanément avec l'étape de déplacement de l'assemblage de source de rayons X (101) pour contrebalancer l'assemblage de source de rayons X (101) ;
**caractérisé en ce que** l'étape de déplacement du contrepoids (111) comprend en outre les étapes suivantes :
le déplacement d'au moins deux masses espacées ; et
le déplacement du centre de masse du contrepoids (111) le long d'une ligne entre lesdites au moins deux masses, colinéairement avec un déplacement du centre de masse de l'assemblage de source de rayons X (101) quand l'assemblage de source de rayons X (101) se déplace le long de la trajectoire d'imagerie.

4. Procédé selon la revendication 3, comprenant en outre le maintien du centre de masse de l'assemblage de source de rayons X (101) et du contrepoids (111) spatialement stationnaire pendant les étapes de déplacement de l'assemblage de source de rayons X (101) et de déplacement du contrepoids (111).

5. Procédé selon la revendication 3, comprenant en outre la rotation de l'assemblage de source de rayons X (101) simultanément avec l'étape de déplacement de l'assemblage de source de rayons X (101) le long de la trajectoire d'imagerie linéaire.
